# FASCICULE DE BREVET EUROPEEN

(11) **EP 0 915 081 B1**
(45) Date de publication et mention de la délivrance du brevet: **22.08.2001**
(21) Numéro de dépôt: 98402693.0
(22) Date de dépôt: 28.10.1998
(51) Int. Cl.: C07C 275/10, C07C 275/16, C07C 275/20, C07F 7/08, A61K 7/06, A61K 7/48

(54) **Composé de type céramide, procédé de préparation et utilisation**
Ceramidverbindungen, Verfahren zu ihrer Herstellung und Verwendung
Ceramide compounds, process for their preparation and use

(30) Priorité: 04.11.1997 FR 9713844
(43) Date de publication de la demande: 12.05.1999
(73) Titulaire: L'OREAL, 75008 Paris (FR)
(72) Inventeur: Tuloup, Pierre, 75014 Paris (FR); Philippe, Michel, 91320 Wissous (FR)
(74) Mandataire: Dodin, Catherine

(56) Documents cités:
- EP-A- 0 383 024
- EP-A- 0 420 722
- DE-A- 2 703 185
- PATENT ABSTRACTS OF JAPAN vol. 013, no. 308 (C-617), 14 juillet 1989 -& JP 01 093562 A (SHIONOGI & CO), 12 avril 1989

## Description

La présente invention a pour objet des composés de type céramide, leur procédé de préparation ainsi que leur utilisation, notamment pour le traitement et le soin de la peau, des cheveux, des ongles et des cils, en cosmétique ou pour la fabrication d'une composition pharmaceutique destinée au traitement et/au soin de la peau et/ou des matières kératiniques.

DE-A-2703185 et EP-A-0383024 décrivent des dérives d'urée ainsi que leurs utilisations en cosmétique. JP-A-01093562 décrit la production de dérivées de la sphingosine possédant des actions anti-tumorales.

L'exposition de la peau au froid, au soleil, aux atmosphères à faible humidité relative, les traitements répétés avec des compositions de lavage ou encore le contact avec des solvants organiques, sont des facteurs qui entraînent, à des degrés divers, un dessèchement apparent. La peau apparaît plus sèche, moins souple et le relief cutané plus prononcé. Par ailleurs, les cheveux, qui sont soumis trop fréquemment à certains traitements capillaires, perdent leur aspect brillant et peuvent devenir rêches et cassants.

Pour résoudre ces problèmes, il a notamment été proposé d'utiliser des céramides. On sait en effet que ces composés sont les éléments constitutifs prépondérants des lipides intercornéocytaires du stratum cornéum et participent au maintien de l'intégrité de la barrière cutanée.
Les céramides utilisés en cosmétique sont le plus souvent des extraits naturels issus notamment de la peau de porc, du cerveau de boeuf, de l'oeuf, des cellules de sang, des végétaux comme le blé, etc. (demandes de brevet japonais J86/260008 et J87/120308). De tels céramides ont été également proposés pour la protection des cheveux (EP 0 278 505).
il s'agit donc toujours de mélanges de teneur plus ou moins importante en céramides et dont la composition est difficile à contrôler. De plus, ces mélanges sont sujets à la contamination bactérienne. Leur conservation est donc difficile à maîtriser.
Lorsqu'ils sont d'origine animale, il y a en plus un risque de contamination par l'agent responsable de la BSE (encéphalopathie bovine spongiforme).

Pour pallier ces inconvénients, il a été proposé des céramides de synthèse, notamment dans les demandes de brevet européen EP500437 et EP647617. Ces composés de l'art antérieur, utilisés dans des compositions cosmétiques ou dermatologiques, pour le traitement et le soin de la peau et des cheveux, ont un effet hydratant permettant de prévenir ou de corriger certains effets du dessèchement apparent de la peau ou des cheveux.

II a également été proposé, dans la demande de brevet européen EP420722, des dérivés lipidiques de céramides répondant à la formule :

R¹ - CH(OH) - CH(CH₂OH) - NH - COOR²

dans laquelle R¹ désigne un radical alcoyle ou alcényle en C₁₁ à C₂₁, et R² désigne un radical hydrocarboné en C₈ à C₃₀.
Dans cet art antérieur, il a été proposé de modifier la jonction amide des céramides, en la remplaçant par une jonction uréthanne. Les composés ainsi obtenus présentent un intérêt particulier pour le traitement de la peau ou des cheveux, et permettent également de prévenir ou de corriger certains effets du dessèchement. Ils présentent par ailleurs une faible agressivité vis-à-vis de la peau ou des muqueuses oculaires, ainsi que des bonnes propriétés émollientes et adoucissantes.

Toutefois, il subsiste toujours le besoin de disposer de composés ayant un effet hydratant et/ou traitant amélioré par rapport aux composés de l'art antérieur, tout en étant susceptibles d'application dans les domaines notamment cosmétique et/ou dermatologique.

La demanderesse a donc recherché des composés qui permettent de prévenir ou de corriger les phénomènes se traduisant par un dessèchement apparent et qui redonnent à la peau sa souplesse et aux cheveux leur brillance et leur douceur.

La présente invention a donc pour objet des composés répondant à la formule (I) :

R¹ - CH(OH) - CH(CH₂OH) - NH - CO - NR²R³

dans laquelle :
* R¹ désigne un radical alkyle, saturé ou insaturé, linéaire ou ramifié, éventuellement hydroxylé, en C₉ à C₂₃
* R² désigne l'hydrogène ou un radical alkyle, saturé ou insaturé, linéaire ou ramifié, en C₁ à C₈
* R³ désigne un radical alkyle, linéaire ou ramifié, saturé ou insaturé, éventuellement hydroxylé, en C₁ à C₃₂, ledit radical hydroxyle pouvant lui-même être substitué par un radical acyle, linéaire ou ramifié, saturé ou insaturé, en C₁ à C₂₄; ledit groupement R³ peut éventuellement être interrompu par 1 7 hétéroatomes.

L'invention a également pour objet un procédé de préparation des composés ci-dessus dans lequel on fait réagir un aminodiol de formule :

R₁ - CH(OH) - CH(NH₂) - CH₂OH

avec un dérivé d'azolide, dans un solvant inerte, de manière à obtenir le composé recherché.

Un autre objet de l'invention est une dispersion aqueuse de sphérules lipidiques constituées de couches moléculaires organisées enfermant une phase aqueuse encapsulée, lesdites couches étant constituées d'au moins un composé de formule (I) ci-dessus associé à au moins un autre composé lipidique.

Un autre objet de l'invention est une composition notamment à usage cosmétique ou pharmaceutique comprenant, dans un milieu cosmétiquement ou pharmaceutiquement acceptable, au moins un composé de formule (I) et/ou une dispersion aqueuse de sphérules lipidiques telle que ci-dessus.

Un autre objet de l'invention est l'utilisation d'un composé de formule (I) ou d'une dispersion aqueuse de sphérules lipidiques le comprenant, en tant qu'agent hydratant, émollient et/ou adoucissant.

Encore un autre objet de l'invention est l'utilisation d'un composé de formule (I) ou d'une dispersion aqueuse de sphérules lipidiques le comprenant, pour le traitement et/ou le soin de la peau et/ou des matières kératiniques, notamment pour le traitement et/ou le soin des peaux abîmées et/ou âgées, et/ou des cheveux et/ou des ongles abîmés, en cosmétique ou pour la fabrication d'une composition pharmaceutique destinée aux dits traitements.

On a constaté que ces composés présentent un très bon pouvoir hydratant de la peau et/ou des matières kératiniques (cheveux, cils, sourcils et ongles). Ils peuvent être utilisés avec un intérêt tout particulier lorsqu'un effet de lutte contre le dessèchement de la peau et/ou des cheveux est recherché en cosmétique ou en pharmaceutique.
Ces composés ont l'avantage d'améliorer et/ou de rétablir la fonction barrière lorsqu'ils sont appliqués sur la peau.
De plus, ces composés présentent une faible agressivité vis-à-vis de la peau ou des muqueuses oculaires, ainsi qu'une bonne tolérance vis-à-vis des membranes cellulaires comme celles des érythrocytes.
Ces composés présentent des propriétés émollientes et adoucissantes, notamment de la peau et des cheveux. Ils peuvent en outre être facilement solubilisés dans les phases grasses des compositions cosmétiques ou pharmaceutiques.
Les composés selon l'invention peuvent être utilisés pour traiter les peaux abîmées et/ou âgées, ainsi que les cheveux ou les ongles abîmés, dans le domaine cosmétique ou pharmaceutique.
Les cheveux traités par ces composés de formule (I) présentent un aspect brillant, un toucher plus doux et une moins grande sensibilité à l'eau, due à l'apport de matière lipidique uniformément répartie sur les écailles du cheveu. Les propriétés mécaniques et de nervosité sont également améliorées.

Les composés selon l'invention répondent donc à la formule suivante :

R¹ - CHOH - CH(CH₂OH) - NH - CO - NR²R³

De préférence, R¹ désigne un radical alkyle linéaire, saturé ou insaturé, éventuellement hydroxylé en C₁₁ à C₁₉.
De préférence, R² désigne l'hydrogène.

De préférence, R³ désigne un radical alkyle linéaire ou ramifié, saturé ou insaturé, en C₄ à C₁₈ pouvant être interrompu par 1 à 5 hétéroatomes. Parmi les hétéroatomes, on peut citer l'oxygène, l'azote et/ou le silicium.

Les composés selon l'invention peuvent être utilisés sous forme d'isomère pur ou de mélanges d'isomères.

Les composés préférés selon l'invention sont :
- le 2-N-(hexadécylureido)-octadécane-1,3-diol,
- le 2-N-(éthyloxycarbonylméthylureido)-octadécane-1,3-diol,
- le 2-N-(oleylureido)-octadécane-1,3-diol,
- le N-(2-amino-octadécane-1,3-diol)-carboxy-N'-aminopropylsiloxane, et
- le 2-N-(dodécylureido)-octadécane-1,3-diol.

Les composés selon l'invention peuvent être aisément préparés par l'homme du métier, notamment à l'aide du procédé objet de l'invention.

Ce procédé consiste à faire réagir un aminodiol de formule :

R₁ - CH(OH) - CH(NH₂) - CH₂OH

avec un dérivé d'azolide, dans un solvant inerte, de manière à obtenir le composé recherché.

De préférence, on utilise un dérivé d'imidazolide tel qu'un imidazolidure.
De préférence, les deux constituants de départ sont ajoutés mole à mole.
De préférence, la réaction est effectuée à chaud, au reflux, pendant 1 à 5 heures, de préférence 2 à 4 heures.
Le solvant inerte est de préférence un solvant organique tel qu'un alcool ou un éther.

Le mélange réactionnel peut, après réaction, être versé dans un bain d'eau glacée, de manière à faire précipiter le composé recherché.
On peut ensuite, de manière usuelle, filtrer, laver et sécher puis, éventuellement, recristalliser ledit composé de formule (I).

Les composés selon l'invention peuvent donc recevoir des applications diverses, notamment dans des compositions cosmétiques ou pharmaceutiques.
Ainsi, les compositions comprenant les composés selon l'invention peuvent notamment être à usage cosmétique ou pharmaceutique. Dans ce cas, elles comprennent, outre le/les composés de formule (I) un milieu cosmétiquement ou pharmaceutiquement acceptable.

Ces compositions peuvent se présenter sous forme d'émulsions (lait ou crème), de lotions hydroalcooliques, huileuses ou oléoalcooliques, de gels, de dispersions ou de bâtonnets solides, de sprays ou de mousse aérosol.
Ces compositions sont par exemple des lotions, des laits ou des crèmes émollients; des laits ou des crèmes pour les soins de la peau ou des cheveux; des crèmes, des lotions ou des laits démaquillants; des bases de fond de teint; des lotions, des laits ou des crèmes antisolaires ou après-soleil; des lotions, des laits ou des crèmes de bronzage artificiel; des crèmes ou des mousses de rasage; des lotions après rasage; des shampooings; des rouges à lèvres; des mascaras éventuellement traitants; des vernis à ongles ou des soins des ongles.
Ces compositions peuvent également se présenter sous la forme de bâtons pour les lèvres destinés soit à les colorer, soit à éviter les gerçures, ou de produits de maquillage pour les yeux ou des fards et fonds de teint pour le visage.

La forme galénique ainsi que la quantité de composé présent dans la composition peuvent être aisément déterminées par l'homme du métier sur la base de ses connaissances générales.

A titre indicatif, le composé de formule (I) peut être présent en une quantité de 0,001 à 20 % en poids par rapport au poids total de la composition, de préférence à raison de 0,01 à 10% en poids.

Lorsque les compositions selon l'invention se présentent sous la forme d'émulsions de type eau-dans-huile ou huile-dans-eau, la phase grasse est de préférence essentiellement constituée d'un mélange de composés de formule (I) avec au moins une huile, et éventuellement un autre corps gras.
La phase grasse des émulsions peut constituer de 5 à 60% du poids total de l'émulsion. La phase aqueuse desdites émulsions constitue de préférence de 30 à 85% du poids total de l'émulsion. La proportion de l'agent émulsionnant peut être comprise entre 1 et 20%, et de préférence entre 2 et 12% du poids total de l'émulsion.

Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau; les lotions huileuses peuvent en outre constituer des huiles moussantes contenant un tensioactif oléosoluble, des huiles pour le bain, etc.

Parmi les principaux adjuvants pouvant être présents dans les compositions selon l'invention, on peut citer les corps gras tels que les huiles ou les cires minérales, animales ou végétales, les acides gras, les esters d'acide gras tels que les triglycérides d'acides gras ayant de 6 à 18 atomes de carbone, les alcools gras; les émulsionnants comme les alcools gras oxyéthylénés ou les alcoyléthers de polyglycérol; les solvants tels que les monoalcools ou polyalcools inférieurs contenant de 1 à 6 atomes de carbone ou encore l'eau.
Les monoalcools ou polyalcools plus particulièrement préférés sont choisis parmi l'éthanol, l'isopropanol, le propylèneglycol, le glycérol et le sorbitol.
A titre de corps gras, parmi les huiles minérales, on peut citer l'huile de vaseline; parmi les huiles animales, les huiles de baleine, de requin, de phoque, de menhaden, de foie de flétan, de morue, de thon, de tortue, de pied de boeuf, de pied de cheval, de pied de mouton, de vison, de loutre, de marmotte, etc.; parmi les huiles végétales, les huiles d'amande, de germes de blé, d'olive, de germe de maïs, de jojoba, de sésame, de tournesol, de palme, de noix, de karité, de shoréa, de macadamia, de pépins de cassis et similaires.
Parmi les esters d'acides gras, on peut utiliser des esters d'acides en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.
On peut également citer comme corps gras, la vaseline, la paraffine, la lanoline, la lanoline hydrogénée, le suif, la lanoline acétylée, les huiles de silicone.
Parmi les cires, on peut citer la cire de Sipol, la cire de lanoline, la cire d'abeille, la cire de Candelilla, la cire microcristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de calcium, magnésium et aluminium.
Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de polyglycérol, les alcools en C₁₂-C₁₈ comportant de 2 à 10 moles de glycérol.

Il peut être aussi utile d'utiliser des épaississants tels que les dérivés de cellulose, les dérivés d'acide polyacrylique, les gommes de guar ou de caroube ou la gomme de xanthane.

Les compositions selon l'invention peuvent également contenir des adjuvants habituellement utilisés en cosmétique ou en pharmacie et notamment des produits hydratants, des adoucissants, des produits pour le traitement d'affections cutanées, des filtres solaires, des germicides, des actifs cosmétiques ou pharmaceutiques, des colorants, des conservateurs, des parfums et des propulseurs.

Lorsque les compositions selon l'invention sont des dispersions, il peut s'agir de dispersions de composés de formule (I) dans l'eau en présence de tensioactif ou encore de dispersions aqueuses de sphérules lipidiques, constituées de couches moléculaires organisées enfermant une phase aqueuse encapsulée, ces couches pouvant être constituées d'au moins un composé de formule (I) associé à au moins un autre composé lipidique.
On a en effet constaté que les composés de formule (I), associés à d'autres lipides, pouvaient être utilisés pour la formation de sphérules lipidiques.

On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les demandes de brevet FR2315991, FR1477048, FR2091516, WO83/01571 et WO92/08685.
On peut par exemple utiliser comme autres lipides, des lipides comportant une chaîne lipophile contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique.
Le groupement hydrophile de ces lipides peut être un groupement ionique ou non-ionique.
A titre de groupements non-ioniques, on peut citer des groupements dérivés de polyéthylèneglycol. On peut aussi utiliser avantageusement comme lipides formant la phase lamellaire, des éthers de polyglycérol tels que ceux décrits dans les brevets français n° 1 477 048, 2 091 516, 2 465 780 et 2 482 128.
A titre de groupement ionique, on peut avantageusement utiliser un groupement dérivé d'un composé amphotère, anionique ou cationique.
D'autres lipides décrits dans la demande de brevet international WO83/01571 comme pouvant être utilisés pour la formation de vésicules sont les glycolipides comme le lactosylcéramide, le galactocérébroside, les gangliosides et le trihexosylcéramide, ainsi que les phospholipides tels que le phosphatidylglycérol et le phosphatidylinositol.

On peut ainsi obtenir une dispersion de sphérules lipidiques constituées de couches moléculaires organisées de composé de formule (I) et de lipide tel que défini ci-dessus renfermant une phase aqueuse à encapsuler.

Les sphérules en dispersion ont généralement-un diamètre compris entre 0,05 µm et 5 µm.

La phase continue de la dispersion qui entoure les sphérules est une phase aqueuse.
La phase aqueuse encapsulée dans les sphérules peut être de l'eau ou une solution aqueuse de substance active et est dans ce cas de préférence isoosmotique par rapport à la phase continue de la dispersion.

Les sphérules peuvent être obtenues en particulier suivant le procédé décrit dans le brevet FR2315991, selon lequel on prépare une dispersion de sphérules constituées de couches moléculaires organisées renfermant une phase aqueuse à encapsuler, en mettant en contact d'une part au moins un composé de formule (I) associé à un ou plusieurs lipides et d'autre part la phase aqueuse à encapsuler dans les sphérules, en agitant pour assurer le mélange et obtenir une phase lamellaire, en ajoutant ensuite un liquide de dispersion en quantité supérieure à la quantité de phase lamellaire obtenue et en secouant énergiquement pendant une durée allant de 15 minutes à 3 heures environ.
Un autre procédé de préparation peut consister à utiliser le procédé dénommé REV (reverse-phase evaporation vesicle) ou évaporation en phase inverse décrit dans Proc. Natl. Acad. Sci. USA., Vol. 75, n° 9, pages 4194-4198 (1978), par Szoka et Papahadjopoulos.
On peut également mettre en oeuvre le procédé qui comprend la succession d'étapes consistant à dissoudre au moins un lipide dans au moins un solvant organique non miscible à l'eau; à ajouter la phase organique ainsi obtenue à une phase aqueuse; à former une dispersion des deux phases sous forte agitation, la taille des vésicules pouvant être réglée en faisant varier la vitesse d'agitation au cours de ce mélange de phase; puis à conduire l'évaporation du/des solvants sous forte agitation; et, le cas échéant, à concentrer la dispersion.

Les substances actives susceptibles d'être présentes dans la phase aqueuse encapsulée peuvent être des substances ayant un intérêt pharmaceutique, alimentaire ou des substances ayant une activité cosmétique. Lorsqu'elles sont hydrosolubles, elles sont dans la phase aqueuse encapsulée à l'intérieur des vésicules. Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels; des agents de brunissage artificiel tels que la dihydroxyacétone, l'érythrulose, le glycéraldéhyde, les γ-dialdéhydes tels que l'aldéhyde tartrique, ces composés étant éventuellement associés à des colorants; des filtres solaires hydrosolubles; des antitranspirants, des déodorants, des astringents, des produits rafraîchissants, toniques, cicatrisants, kératolytiques, dépilatoires, des eaux parfumées; des extraits de tissus végétaux, tels que les polysaccharides; des colorants hydrosolubles; des agents antipelliculaires; des agents antiséborrhéiques; des oxydants tels que des agents de décoloration comme l'eau oxygénée; des réducteurs tels que l'acide thioglycolique et ses sels.
On peut citer également les vitamines, les hormones, les enzymes telles que la superoxyde dismutase, les vaccins, les anti-inflammatoires tels que l'hydrocortisone, les antibiotiques, les bactéricides, les agents cytotoxiques ou anti-tumoraux.
Lorsque les substances actives sont liposolubles, elles se trouvent incorporées dans les feuillets des vésicules. Elle peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

Les dispersions de sphérules lipidiques présentent l'intérêt de véhiculer des substances actives qui se trouvent ainsi masquées et protégées vis-à-vis des différents agents d'altération : oxydants et plus généralement composés réactifs vis-à-vis des substances actives encapsulées. La pénétration et la fixation des substances actives peuvent être modulées par la variation de la taille des sphérules et de leur charge électrique. L'action de ces substances actives peut également être ainsi différée (effet retard).

Ces dispersions aqueuses de sphérules constituées de couches moléculaires organisées de composés de formule (I) associés à d'autres lipides renfermant une phase aqueuse à encapsuler, peuvent notamment être utilisées dans les domaines cosmétique ou pharmaceutique, en particulier pour le traitement et/ou le soin de la peau et des matières kératiniques telles que le cheveu, les cils, les sourcils et les ongles.
Elles peuvent être utilisées telles quelles ou introduites dans une composition cosmétique ou pharmaceutique.

L'invention est illustrée plus en détail dans les exemples suivants.

### Exemples de préparation de composés selon l'invention

Les composés selon l'invention peuvent être préparés en faisant réagir les constituants suivants :

C₁₅H₃₁ - CH(OH) - CH(CH₂OH) - NH₂

et 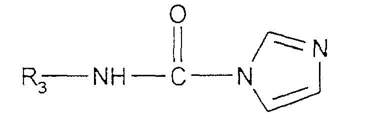 de manière à obtenir un composé selon l'invention dans lequel :
- R₁ est un radical alkyle saturé ayant 15 atomes de carbone,
- R₂ est l'hydrogène

Le mode opératoire général consiste à additionner un équivalent de dérivé d'imidazolide (classiquement préparé par réaction de carbonyldiimidazole sur une amine) et un équivalent de 2-amino-octadécane-1,3-diol dissous dans le minimum d'isopropanol.
On chauffe à reflux pendant 3 heures puis on verse le mélange réactionnel dans un bain d'eau glacée.
Le composé recherché précipite. Il est filtré, lavé et séché puis, éventuellement, recristallisé dans l'éthanol.

### Exemple 1 : Préparation du 2-N-(hexadecvluréido)-octadécane-1,3-diol

Le radical R₃ est un radical -C₁₆H₃₃.
On obtient, après filtration, séchage et recristallisation, un solide blanc avec un rendement de 76%.

Point de fusion : environ 92°C

| Analyse élémentaire : | | | | |
|---|---|---|---|---|
| | C | H | N | O |
| % calculé | 73,89 | 12,76 | 4,92 | 8,44 |
| % trouvé | 74,07 | 12,73 | 4,83 | 8,22 |

L'analyse élémentaire du produit obtenu est conforme au produit attendu.

### Exemple 2 : Préparation du 2-N-(dodécyluréido)-octadécane-1,3-diol

Le radical R₃ est un radical -C₁₂H₂₅.
On obtient, après filtration, séchage et recristallisation, un solide blanc avec un rendement de 57%.

Point de fusion : environ 81,8°C

| Analyse élémentaire : | | | | |
|---|---|---|---|---|
| | C | H | N | O |
| % calculé | 72,60 | 12,58 | 5,46 | 9,36 |
| % trouvé | 72,00 | 12,29 | 5,51 | 9,78 |

L'analyse élémentaire du produit obtenu est conforme au produit attendu.

### Exemple 3 : Préparation du 2-N-(éthyloxycarbonylméthyluréido)-octadécane-1,3-diol

Le radical R₃ est un radical -CH₂-COO-C₂H₅.
On obtient, après filtration et séchage, un composé solide avec un rendement de 76%.

| Analyse élémentaire : | | | | |
|---|---|---|---|---|
| | C | H | N | O |
| % calculé | 64,15 | 10,77 | 6,51 | 18,58 |
| % trouvé | 61,84 | 10,79 | 6,52 | 17,78 |

L'analyse élémentaire du produit obtenu est conforme au produit attendu.

### Exemple 4 : Préparation du N-(2-amino-octadécane-1,3-diol)-carboxy-N'-aminopropylsiloxane.

Le radical R₃ est un radical :

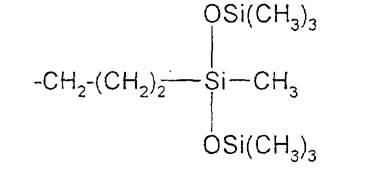

On obtient, après filtration et séchage, un solide blanc avec un rendement de 85%.

| Analyse élémentaire : | | | | | |
|---|---|---|---|---|---|
| | C | H | N | O | Si |
| % calculé | 57,37 | 10,96 | 4,61 | 13,18 | 13,88 |
| % trouvé | 57,85 | 10,89 | 14,89 | - | 13,40 |

L'analyse élémentaire du produit obtenu est conforme au produit attendu.

### Exemple 5 : Préparation du 2-N-(oleyluréido)-octadécane-1,3-diol

Le radical R₃ est un radical -CH₂-C₇H₁₄-CH=CH-C₈H₁₇
On obtient, après filtration, séchage et recristallisation, un composé blanc solide avec un rendement de 51%.

| Analyse élémentaire : | | | | |
|---|---|---|---|---|
| | C | H | N | O |
| % calculé | 74,69 | 12,54 | 4,71 | 8,07 |
| % trouvé | 72,94 | 12,70 | 4,71 | 8,04 |

L'analyse élémentaire du produit obtenu est conforme au produit attendu.

### Exemple 6 : Mesure de la perte insensible en eau (PIE)

Cette mesure s'effectue à l'aide d'un évaporimètre (Servomed) qui détermine quantitativement l'évaporation d'eau, c'est-à-dire un transport d'eau par diffusion, à partir d'un échantillon de stratum cornéum préalablement délipidé obturant une capsule cylindrique contenant de l'eau, le tout étant placé dans une chambre à température et humidité relatives contrôlées.
Des capteurs permettent de mesurer la pression partielle de vapeur d'eau en deux points situés à des distances différentes de l'échantillon.
On détermine ainsi le gradient de pression partielle de vapeur d'eau entre les deux points, et donc le taux d'évaporation conformément à la loi de Fick.

Il a été réalisé un test comparatif des effets sur la P.I.E. de solution à 1% du composé à tester dans un mélange de solvant dichlorométhane/méthanol en une proportion 2/1.
Le test a été effectué à 30°C et sous une humidité relative de 40%.
Les composés testés sont :
- composé A : le 2-N-(hexadécylureido)-octadécane-1,3-diol (exemple 1 de l'invention)
- composé B : le N-hexadécyloxycarbonyl-2-amino-octadécane-1,3-diol (exemple 2 de EP420722, art antérieur proche)

Les résultats sont rassemblés dans le tableau suivant.

| Composé | Concentration | P.I.E. à 20 heures (%) |
|---|---|---|
| composé A | 1% | -26 ± 2 |
| composé B | 1% | -16 ± 4 |

On constate donc que l'application des composés selon l'invention permet de réduire de manière significative l'évaporation de l'eau contenue dans le stratum cornéum, démontrant ainsi les propriétés améliorées pour les composés selon l'invention de barrière de perméabilité à l'eau de stratum cornéum.

### Exemple 7 : composition d'après-shampooing

Un après-shampooing pour le soin ou le traitement des cheveux est préparé à l'aide des constituants suivants :
- alcool cétylstéarylique 2,5 g
- mélange myristate/palmitate/stéarate de myristyle/cétyle/stéaryle 0,5 g
- chlorure de behenyltriméthylammonium à 80% dans un mélange eau/isopropanol (Catinal DC 80 de TOHO) 0,6 g
- PDMS à groupements aminoéthylaminopropyl en émulsion à 35% dans l'eau (DC939 de Dow Corning) 4 g
- composé de l'exemple 1 0,6 g
- conservateurs, parfum qs
- eau qsp 100 g

### Exemple 8 : composition de shampooing

Un shampooing pour le soin ou le traitement des cheveux est préparé à l'aide des constituants suivants :
- lauryl éther sulfate de sodium 16 g MA
- cocoylbétaïne 2,5 g MA
- hydroxypropylcellulose réticulée à l'épichlorhydrine quaternisé par la triméthylamine 0,2 g
- composé de l'exemple 1 0,1 g
- conservateurs, parfum qs
- HCl - qs pH 6,5
- eau qsp 100 g

### Exemple 9 : composition de shampooing

Un shampooing pour le soin ou le traitement des cheveux est préparé à l'aide des constituants suivants :
- lauryl éther sulfate de sodium 16 g MA
- cocoylbétaïne 2,5 g MA
- hydroxypropylcellulose réticulée à l'épichlorhydrine quaternisé par la triméthylamine 0,2 g
- composé de l'exemple 2 0,1 g
- conservateurs, parfum qs
- HCI qs pH 6,5
- eau qsp 100 g

### Exemple 10 : huile de soin pour le corps

Une huile hydratante pour peaux sèches est préparée à l'aide des constituants suivants :
- triglycérides caprylique/caprique 6,5 g
- propylène glycol dicaprylate/dicaprate 22 g
- octanoate de cétéaryle et myristate d'isopropyle 5 g
- néopentanoate d'isostéaryle 2,5 g
- huile d'arachide 5,25 g
- composé de l'exemple 5 1,5 g
- antioxydant, conservateurs, parfum qs
- cyclométhicone qsp 100 g

### Exemple 11 : crème de soin du visage

Une crème de soin hydratante pour peaux normales et mixtes est préparée à l'aide des constituants suivants :
- pétrolatum 4 g
- polyisobutène hydrogéné 6,5 g
- cétyl alcool 2,7 g
- tristéarate de sorbitan 0,5 g
- stéarate de PEG 40 3,2 g
- myristate de myristyle 3 g
- stéarate de glycéryle 3 g
- beurre de karité 2 g
- cyclométhicone 5 g
- acide stéarique 0,2 g
- hydroxyde de sodium 0,5 g
- citrate de sodium 0,1 g
- composé de l'exemple 1 1 g
- antioxydant, conservateurs, parfum qs
- eau qsp 100 g

### Exemple 12 : crème nourrissante pour le visage

Une crème de soin pour peaux sèches et mixtes est préparée à l'aide des constituants suivants :
- cétyl diméthicone copolyol 2,5 g
- polyglycéryle-4 isostéarate 1,5 g
- isoparaffine 10 g
- huile de noyaux d'abricot 5 g
- cyclométhicone 8 g
- acetylated glycol stéarate et tristéarine 1 g
- glycérine 5 g
- sulfate de magnésium 0,7 g
- aluminium starch octénylsuccinate 3 g
- composé de l'exemple 3 1 g
- antioxydant, conservateurs, parfum qs
- eau qsp 100 g

### Exemple 13 : crème de soin pour le visage

Une crème de jour est préparée à l'aide des constituants suivants
- stéarate de sucrose 5 g
- méthyl glucose sesquistéarate 2,5 g
- huile de noyaux d'abricot 11 g
- acide stéarique 1 g
- gélifiant 0,4 g
- cyclométhicone 9 g
- isostéarate d'isopropyle 5 g
- composé de l'exemple 1 1 g
- antioxydant, conservateurs, parfum qs
- eau qsp 100 g

### Exemple 14 : crème de soin du visage

Une crème de soin hydratante est préparée à l'aide des constituants suivants :
- tristéarate de sorbitan 0,25 g
- stéarate de PEG 40 4 g
- cétyl alcool 1,5 g
- myristate de myristyle - 2 g
- stéarate de glycéryle 3 g
- octyl palmitate 2 g
- polyisobutène hydrogéné 13,5 g
- cyclométhicone 10 g
- glycérine 6 g
- composé de l'exemple 2 0,8 g
- antioxydant, conservateurs, parfum qs
- eau qsp 100 g

## Revendications

1. Composé répondant à la formule (I):
R¹ - CH(OH) - CH(CH₂OH) - NH - CO - NR²R³
dans laquelle :
* R¹ désigne un radical alkyle, saturé ou insaturé, linéaire ou ramifié, éventuellement hydroxylé, en C₉ à C₂₃
* R² désigne l'hydrogène ou un radical alkyle, saturé ou insaturé, linéaire ou ramifié, en C₁ à C₈
* R³ désigne un radical alkyle, linéaire ou ramifié, saturé ou insaturé, éventuellement hydroxylé, en C₁ à C₃₂, ledit radical hydroxyle pouvant lui-même être substitué par un radical acyle, linéaire ou ramifié, saturé ou insaturé, en C₁ à C₂₄; ledit groupement R³ peut éventuellement être interrompu par 1 à 7 hétéroatomes.

2. Composé selon la revendication 1, dans lequel le radical R¹ désigne un radical alkyle linéaire, saturé ou insaturé, éventuellement hydroxylé, en C₁₁ à C₁₉; et/ou le radical R² désigne l'hydrogène; et/ou le radical R³ désigne un radical alkyle linéaire ou ramifié, saturé ou insaturé, en C₄ à C₁₈ pouvant être interrompu par 1 à 5 hétéroatomes.

3. Composé selon la revendication 1, choisi parmi :
- le 2-N-(hexadécylureido)-octadécane-1,3-diol,
- le 2-N-(éthyloxycarbonylméthylureido)-octadécane-1,3-diol,
- le 2-N-(oleylureido)-octadécane-1,3-diol,
- le N-(2-amino-octadécane-1,3-diol)-carboxy-N'-aminopropylsiloxane, et
- le 2-N-(dodécylureido)-octadécane-1,3-diol.

4. Procédé de préparation des composés selon l'une des revendications 1 à 3, dans lequel on fait réagir un aminodiol de formule:
R₁ - CH(OH) - CH(NH₂) - CH₂OH
avec un dérivé d'azolide, dans un solvant inerte, de manière à obtenir le composé recherché.

5. Dispersion aqueuse de sphérules lipidiques constituées de couches moléculaires organisées enfermant une phase aqueuse encapsulée, lesdites couches étant constituées d'au moins un composé de formule (I) selon l'une des revendications 1 à 3, associé à au moins un autre composé lipidique.

6. Composition notamment à usage cosmétique ou pharmaceutique comprenant, dans un milieu cosmétiquement ou pharmaceutiquement acceptable, au moins un composé de formule (I) selon l'une des revendications 1 à 3 et/ou une dispersion aqueuse de sphérules lipidiques selon la revendication 5.

7. Composition selon la revendication 6, dans laquelle le composé de formule (I) est présent en une quantité de 0,001 à 20 % en poids par rapport au poids total de la composition, de préférence à raison de 0,01 à 10% en poids.

8. Composition selon l'une des revendications 6 à 7, comprenant au moins un adjuvant choisi parmi les corps gras, les solvants, l'eau, les épaississants, les émulsionnants, les produits hydratants, les adoucissants, les filtres solaires, les germicides, les actifs cosmétiques ou pharmaceutiques, les colorants, les conservateurs, les parfums, les propulseurs et les tensioactifs.

9. Composition selon l'une des revendications 6 à 8, se présentant sous forme d'émulsion, de lotion hydroalcoolique, huileuse ou oléoalcoolique, de gel, de dispersion ou de bâtonnet solide, de spray ou de mousse aérosol.

10. Utilisation d'un composé de formule (I) selon l'une des revendications 1 à 3, ou d'une dispersion aqueuse de sphérules lipidiques selon la revendication 5, en tant qu'agent hydratant, émollient et/ou adoucissant.

11. Utilisation d'un composé de formule (I) selon l'une des revendications 1 à 3, ou d'une dispersion aqueuse de sphérules lipidiques selon la revendication 5, dans une composition cosmétique, pour le traitement et/ou le soin de la peau et/ou des matières kératiniques.

12. Utilisation d'un composé de formule (I) selon l'une des revendications 1 à 3, ou d'une dispersion aqueuse de sphérules lipidiques selon la revendication 5, pour la fabrication d'une composition pharmaceutique destinée au traitement et/ou au soin de la peau et/ou des matières kératiniques.

13. Utilisation selon l'une des revendications 11 à 12, pour le traitement et/ou le soin des peaux abîmées et/ou âgées, et/ou des cheveux et/ou des ongles abîmés.

14. Utilisation d'au moins un composé de formule (I) selon l'une des revendications 1 à 3, associé à au moins un autre lipide, pour la formation de sphérules lipidiques.

## Patentansprüche

1. Verbindung der Formel (I):
R¹ - CH(OH) - CH(CH₂OH) - NH - CO - NR² R³
worin bedeuten:
- R¹ eine gesättigte oder ungesättigte, geradkettige oder verzweigte, gegebenenfalls hydroxylierte C₉₋₂₃-Alkylgruppe,
- R² Wasserstoff oder eine gesättigte oder ungesättigte, geradkettige oder verzweigte C₁₋₈-Alkylgruppe und
- R³ eine geradkettige oder verzweigte, gesättigte oder ungesättigte, gegebenenfalls hydroxylierte C₁₋₃₂-Alkylgruppe, wobei die Hydroxygruppe mit einer geradkettigen oder verzweigten, gesättigten oder ungesättigten C₁₋₂₄-Acylgruppe substituiert sein kann, wobei die Gruppe R³ gegebenenfalls durch 1 bis 7 Heteroatome unterbrochen sein kann.

2. Verbindung nach Anspruch 1, worin die Gruppe R¹ eine geradkettige, gesättigte oder ungesättigte, gegebenenfalls hydroxylierte C₁₁₋₁₉-Alkylgruppe und/oder die Gruppe R² Wasserstoff und/oder die Gruppe R³ eine geradkettige oder verzweigte, gesättigte oder ungesättigte C₄₋₁₈-Alkylgruppe, die durch 1 bis 5 Heteroatome unterbrochen sein kann, bedeuten.

3. Verbindung nach Anspruch 1, die ausgewählt ist unter:
- 2-N-(Hexadecylureido)-octadecan-1,3-diol,
- 2-N-(Ethyloxycarbonylmethylureido)-octadecan-1,3-diol,
- 2-N-(Oleylureido)-octadecan-1,3-diol,
- N-(2-Amino-octadecan-1,3-diol)-carboxy-N'-aminopropylsiloxan und
- 2-N-(Dodecylureido)-octadecan-1,3-diol.

4. Verfahren zur Herstellung von Verbindungen nach einem der Ansprüche 1 bis 3, worin in einern inerten Lösemittel ein Aminodiol der Formel:
R₁ - CH(OH) - CH(NH₂) - CH₂OH
mit einem Azolidderivat umgesetzt wird, um die gewünschte Verbindung zu erhalten.

5. Wässerige Dispersion von Lipidkügelchen, die aus organisierten molekularen Schichten bestehen, die eine eingekapselte wässerige Phase umschließen, wobei die Schichten aus mindestens einer Verbindung der Formel (I) nach einem der Ansprüche 1 bis 3 in Kombination mit mindestens einer weiteren Lipidverbindung bestehen.

6. Zusammensetzung insbesondere zur kosmetischen oder pharmazeutischen Anwendung, die in einem kosmetisch oder pharmazeutisch akzeptablen Medium mindestens eine Verbindung der Formel (I) nach einem der Ansprüche 1 bis 3 und/oder eine wässerige Dispersion von Lipidkügelchen nach Anspruch 5 enthält.

7. Zusammensetzung nach Anspruch 6, worin die Verbindung der Formel (I) in einem Mengenanteil von 0,001 bis 20 Gew.-% und vorzugsweise von 0,01 bis 10 Gew.-%, bezogen auf das Gesamtgewicht der Zusammensetzung, vorliegt.

8. Zusammensetzung nach einem der Ansprüche 6 bis 7, die mindestens einen Zusatzstoff enthält, der unter Fettsubstanzen, Lösemitteln, Wasser, Verdickungsmitteln, Emulgatoren, hydratisierenden Produkten, reizlindernden Mitteln, Sonnenschutzfiltern, Germiziden, kosmetischen oder pharmazeutischen Wirkstoffen, Färbemitteln, Konservierungsstoffen, Parfums, Treibmitteln und grenzflächenaktiven Stoffen ausgewählt ist.

9. Zusammensetzung nach einem der Ansprüche 6 bis 8, die als Emulsion, als wässerig-alkoholische, ölige oder ölig-alkoholische Lotion, als Gel, Dispersion oder als fester Stift, als Spray oder als Aerosolschaum vorliegt.

10. Verwendung einer Verbindung der Formel (I) nach einem der Ansprüche 1 bis 3 oder einer wässerigen Dispersion von Lipidkügelchen nach Anspruch 5 als Hydratisierungsmittel, Emollens und/ oder reizlinderndes Mittel.

11. Verwendung einer Verbindung der Formel (I) nach einem der Ansprüche 1 bis 3 oder einer wässerigen Dispersion von Lipidkügelchen nach Anspruch 5 in einer kosmetischen Zusammensetzung zur Behandlung und/oder Pflege der Haut und/oder keratinischer Materialien.

12. Verwendung einer Verbindung der Formel (I) nach einem der Ansprüche 1 bis 3 oder einer wässerigen Dispersion von Lipidkügelchen nach Anspruch 5 zur Herstellung einer pharmazeutischen Zusammensetzung, die zur Behandlung und/oder Pflege der Haut und/oder keratinischer Materialien bestimmt ist.

13. Verwendung nach einem der Ansprüche 11 bis 12 zur Behandlung und/oder Pflege geschädigter und/oder reifer Haut und/oder geschädigter Haare und/oder Nägel.

14. Verwendung mindestens einer Verbindung der Formel (I) nach einem der Ansprüche 1 bis 3 in Kombination mit mindestens einem weiteren Lipid zur Bildung von Lipidkügelchen.

## Claims

1. Compound corresponding to the formula (I) :
R¹-CH(OH)-CH(CH₂OH)-NH-CO-NR²R³
in which:
R¹ denotes an optionally hydroxylated, saturated or unsaturated, linear or branched, C₉ to C₂₃ alkyl radical
R² denotes hydrogen or a saturated or unsaturated, linear or branched, C₁ to C₈ alkyl radical
R³ denotes an optionally hydroxylated, saturated or unsaturated, linear or branched, C₁ to C₃₂ alkyl radical, it being possible for the said hydroxyl radical itself to be substituted by a saturated or unsaturated, linear or branched, C₁ to C₂₄ acyl radical; the said R³ group can optionally be interrupted by 1 to 7 heteroatoms.

2. Compound according to Claim 1, in which the R¹ radical denotes an optionally hydroxylated, saturated or unsaturated, linear C₁₁ to C₁₉ alkyl radical; and/or the R² radical denotes hydrogen; and/or the R³ radical denotes a saturated or unsaturated, linear or branched, C₄ to C₁₈ alkyl radical which can be interrupted by 1 to 5 heteroatoms.

3. Compound according to Claim 1, chosen from:
- 2-(N-hexadecylureido)octadecane-1,3-diol,
- 2-(N-ethyloxycarbonylmethylureido)octadecane-1,3-diol,
- 2-(N-oleylureido)octadecane-1,3-diol,
- N-(2-aminooctadecane-1,3-diol)carboxy-N'-aminopropylsiloxane, and
- 2-(N-dodecylureido)octadecane-1,3-diol.

4. Process for the preparation of the compounds according to one of Claims 1 to 3, in which an aminodiol of formula:
R₁-CH(OH)-CH(NH₂)-CH₂OH
is reacted with an azolide derivative in an inert solvent, so as to obtain the desired compound.

5. Aqueous dispersion of lipid spherules composed of organized molecular layers enclosing an encapsulated aqueous phase, the said layers being composed of at least one compound of formula (I) according to one of Claims 1 to 3 in combination with at least one other lipid compound.

6. Composition, in particular for cosmetic or pharmaceutical use, comprising, in a cosmetically or pharmaceutically acceptable medium, at least one compound of formula (I) according to one of Claims 1 to 3 and/or one aqueous dispersion of lipid spherules according to Claim 5.

7. Composition according to Claim 6, in which the compound of formula (I) is present in an amount of 0.001 to 20% by weight with respect to the total weight of the composition, preferably in a proportion of 0.01 to 10% by weight.

8. Composition according to either of Claims 6 and 7, comprising at least one adjuvant chosen from fatty substances, solvents, water, thickening agents, emulsifying agents, moisturizing products, softeners, sunscreen agents, germicides, cosmetic or pharmaceutical active principles, colorants, preservatives, fragrances, propellants and surfaetants.

9. Composition according to one of Claims 6 to 8, which is provided. in the form of an emulsion, an aqueous/alcoholic, oily or oleoalcoholic lotion, a gel, a solid- stick or dispersion, or an aerosol foam or spray.

10. Use of a compound of formula (I) according to one of Claims 1 to 3 or of an aqueous dispersion of lipid spherules according to Claim 5 as moisturizing, emollient and/or softening agent.

11. Use of a compound of formula (I) according to one of Claims 1 to 3 or of an aqueous dispersion of lipid spherules according to Claim 5 in a cosmetic composition for treating and/or caring for the skin and/or keratinous substances.

12. Use of a compound of formula (I) according to one of Claims 1 to 3 or of an aqueous dispersion of lipid spherules according to Claim 5 for the manufacture of a pharmaceutical composition intended for treating and/or caring for the skin and/or keratinous substances.

13. Use according to either of Claims 11 and 12 for treating and/or caring for damaged and/or aged skin and/or damaged hair and/or nails.

14. Use of at least one compound of formula (I) according to one of Claims 1 to 3, in combination with at least one other lipid, for the formation of lipid spherules.
